# EUROPÄISCHE PATENTANMELDUNG

(11) **EP 3 756 718 A1**
(43) Veröffentlichungstag der Anmeldung: **30.12.2020**
(21) Anmeldenummer: 20180023.2
(22) Anmeldetag: 15.06.2020
(51) Int. Cl.: A61M 16/08, A61M 16/10, A61M 16/00

(54) **KONNEKTOR MIT EINER AUFNAHME FÜR EINEN SENSOR**

(30) Priorität: 28.06.2019 DE 102019004508
(71) Anmelder: Drägerwerk AG & Co. KGaA, 23558 Lübeck (DE)
(72) Erfinder: Tappehorn, Ludger, 23558 Lübeck (DE); Lütkhoff, Jan-Henning, 23558 Lübeck (DE)

(57) **Zusammenfassung**

Die vorliegende Erfindung betrifft einen Konnektor (100) zum Anordnen mindestens eines Sensors an einem Beatmungssystem. Der Konnektor umfasst mindestens einen Grundkörper (103) zur Verbindung mit mindestens einem Ventilationsgerät, mindestens ein Schwenkelement (101) zur Verbindung mit einem Nutzer, und mindestens eine Aufnahme (125) für mindestens einen Sensor,
wobei das mindestens eine Schwenkelement (101) auf einer ersten Seite (119) direkt und nicht lösbar mit dem Grundkörper (103) verbunden ist, und wobei das mindestens eine Schwenkelement (101) um mindestens eine Achse gegenüber zumindest einem Teil des Grundkörpers (103) schwenkbar ist, um den Grundkörper (103) gegenüber durch den Nutzer vermittelten Zug- bzw. Druckkräften zu schützen.

## Beschreibung

Die Erfindung betrifft einen Konnektor zum Verbinden mindestens eines Ventilationsgeräts mit einem Nutzer und einen Konnektor zur Verwendung bei einer Beatmung eines Nutzers.

### STAND DER TECHNIK

Zur optimalen Behandlung bzw. Versorgung von beatmungspflichtigen Nutzern, wie bspw. Patienten, sind Beatmungs- bzw. Ventilationsgeräte in der Regel mit unterschiedlichen Möglichkeiten zur Erfassung nutzerabhängiger Parameter ausgestattet. Dazu gehören bspw. eine Volumenstrommessung zur Bestimmung eines Atemminutenvolumens, eine Druckmessung zur Bestimmung eines Atemwegdrucks sowie eine Messfunktion zur Bestimmung einer Kohlenstoffdioxidkonzentration im Atemgas.

Insbesondere die Bestimmung der Kohlenstoffdioxidkonzentration gibt einem Anwender, wie bspw. einem Arzt, wichtige Informationen über einen Zustand bzw. einen Metabolismus eines Nutzers, wie bspw. einem Patienten.

Zur Messung des Atemminutenvolumens und der Kohlenstoffdioxidkonzentration ist es bekannt, einen Sensor als zusätzliche Komponente in eine Verbindungsleitung zwischen einem Ventilationsgerät und einem Nutzer einzubringen, wodurch sich ein Totraum eines resultierenden Gesamtsystems erhöht.

Als Totraum bezeichnet man in diesem Zusammenhang den Teil eines Beatmungssystems, der während einer Exspiration und während einer Inspiration mit Atemgas durchströmt wird.

Beim Beatmen eines Nutzers werden in der Regel Konnektoren eingesetzt, die einen Luftstrom von einem Ventilationsgerät zu dem Nutzer leiten. Derartige Konnektoren können bspw. als Winkelkonnektoren ausgestaltet sein, um eine ergonomische Anordnung an dem Nutzer zu ermöglichen.

Bei der Verwendung eines Konnektors zur künstlichen Beatmung eines Nutzers kommt es in der Regel zu Situationen, in denen der Konnektor, bedingt durch Bewegungen des Nutzers, mit einer Zug- und/oder Druckkraft beaufschlagt wird.

Aus der US 2015/040898 A1 ist ein Konnektor bekannt, der eine Drehbarkeit eines Schlauchsystems gewährleistet. Dabei ist der Konnektor als modulare Einheit ausgestaltet, die jeweils auf einer Nutzerseite und einer Geräteseite mit einer Schnittstelle versehen ist, an der zusätzliche Geräte angeordnet werden können.

Aus der US 2009/320846 A1 der Firma Drägerwerk AG & Co. KGaA ist eine Küvette mit zwei gegenüberliegenden Messfenstern zum Erfassen einer Kohlenstoffdioxidkonzentration in einem durch ein Beatmungssystem strömenden Luftstrom beschrieben.

Durch eine modulare bzw. serielle Anordnung von Sensoren, d.h. eine Aneinanderreihung von Sensoren und Konnektoren, kommt es in einem Beatmungssystem in der Regel zu einer Totraumerhöhung und/oder einer Beeinflussung von Messwerten durch zusätzliche Komponenten, wie bspw. Filter, in einem zu messenden Luftstrom.

### OFFENBARUNG DER ERFINDUNG

Ausgehend von diesem Stand der Technik hat der Erfindung die Aufgabe zugrunde gelegen, einen Konnektor zum Verbinden mindestens eines Ventilationsgeräts mit einem Nutzer bereitzustellen, der diese Nachteile zumindest teilweise überwindet. Es ist daher die Aufgabe der vorliegenden Erfindung, einen Konnektor bereitzustellen, der ein sicheres und genaues Überprüfen einer Qualität eines Luftstroms zum Beatmen eines Nutzers ermöglicht.

Die voranstehende Aufgabe wird gelöst durch den jeweiligen Gegenstand der unabhängigen Ansprüche. Weitere Merkmale und Details der Erfindung ergeben sich aus den Unteransprüchen, der Beschreibung und den Zeichnungen.

Es wird somit ein Konnektor zum Anordnen mindestens eines Sensors an einem Beatmungssystem, vorgestellt. Der Konnektor umfasst mindestens einen Grundkörper zur Verbindung mit mindestens einem Ventilationsgerät, mindestens ein Schwenkelement zur Verbindung mit einem Nutzer, und mindestens eine Aufnahme für mindestens einen Sensor. Das mindestens eine Schwenkelement ist auf einer ersten Seite direkt bzw. unlösbar mit dem Grundkörper verbunden und um mindestens eine Achse gegenüber zumindest einem Teil des Grundkörpers schwenkbar, um den Grundkörper gegenüber durch den Nutzer vermittelten Zug- bzw. Druckkräften zu schützen.

Unter einer direkten Verbindung ist im Kontext der vorliegenden Offenbarung eine Verbindung ohne ein weiteres zwischengeordnetes Element zu verstehen. Durch eine direkte Verbindung zwischen einem ersten Element und einem zweiten Element ist eine Reihenfolge einer das erste Element und das zweite Element umfassenden Anordnung, bei der das erste Elemente auf das zweite Element unmittelbar folgt, zwingend vorgegeben.

Unter einer Aufnahme ist im Kontext der vorgestellten Erfindung ein Verbindungselement zu verstehen, das einem Sensor eine Messung eines durch den erfindungsgemäßen Konnektor strömenden Luftstroms an einer fest vorgegebenen Stelle an dem Konnektor ermöglicht. Eine Aufnahme kann einen Sensor mit dem Konnektor direkt verbinden, indem der Sensor in die Aufnahme eingebracht wird, oder den Sensor bspw. über mindestens ein Verbindungselement mit einer Drucksäule versorgen.

Der vorgestellte Konnektor dient insbesondere zum Bestimmen einer Qualität eines zum Beatmen eines Nutzers bereitgestellten Luftstroms bei einem minimalen Totraum. Dazu ist vorgesehen, dass das erfindungsgemäß vorgesehene mindestens eine Schwenkelement auf einer ersten Seite direkt und unlösbar jedoch schwenkbar mit dem mindestens einen erfindungsgemäß vorgesehenen Grundkörper verbunden ist, so dass das Schwenkelement in einer vorgegebenen Position relativ zu dem Grundkörper angeordnet ist und somit insbesondere lediglich ein Freiheitsgrad bezüglich der relativen Position des Schwenkelements zu dem Grundkörper durch die Schwenkbarkeit, nicht jedoch durch eine Positionsänderung besteht. Insbesondere kann das Schwenkelement als in mehrere Richtungen bzw. in mehreren Freiheitsgraden schenkbares Kugelgelenk ausgestaltet sein. Entsprechend verhindert die direkte Verbindung des Schwenkelements mit dem Grundkörper eine Anordnung weiterer Komponenten, wie bspw. Filtern, zwischen dem Schwenkelement und dem Grundkörper, so dass ein Volumen des Konnektors unabhängig von einer Position in einem jeweiligen Beatmungssystem konstant ist.

Der Grundkörper bildet mit dem Schwenkelement eine Einheit, indem der Grundkörper mit dem Schwenkelement nicht lösbar, jedoch schwenkbar verbunden ist.

Der erfindungsgemäße Konnektor umfasst mindestens eine Aufnahme für mindestens einen Sensor, die insbesondere an dem Schwenkelement angeordnet sein kann. Dies bedeutet, dass durch die vorgegebene relative Position des Schwenkelements gegenüber dem Grundköper auch ein mit dem Schwenkelement über die Aufnahme verbundener Sensor sich an einer vorgegebenen relativen Position gegenüber dem Grundkörper befindet. Mit anderen Worten bewirkt der erfindungsgemäße Konnektor eine vorgegebene Positionierung des Schwenkelements, des Grundkörpers und des Sensors relativ zueinander.

Insbesondere kann der Grundkörper direkt oder über ein Verbindungselement mit einem Ventilationsgerät verbunden sein und, dadurch bedingt, ein Beatmungssystem bilden.

Insbesondere kann das Schwenkelement auf einer zweiten Seite direkt, bspw. unter Verwendung eines Mundstücks oder einem Tubus, mit einem Nutzer verbunden sein. Dazu kann das Schwenkelement konische Anschlüsse, bspw. gemäß der ISO-Norm 5356 aufweisen.

Das erfindungsgemäß vorgesehene Schwenkelement ist auf seiner Nutzerseite insbesondere so nahe wie möglich am Nutzer angeordnet, um einen Totraum eines entsprechenden Gesamtsystems zu minimieren. Es kann vorgesehen sein, dass zwischen Schenkelement und Nutzer ein Modul, wie bspw. ein Filter angeordnet ist.

Insbesondere kann die Aufnahme des Schwenkelements konische Anschlüsse, bspw. gemäß der ISO-Norm 5356, zur Verbindung mit einem Sensor aufweisen.

Das erfindungsgemäß vorgesehene Schwenkelement ermöglicht eine Reduktion von durch einen Nutzer vermittelten Zug- und/oder Druckkräften auf den erfindungsgemäßen Konnektor, die bspw. durch Bewegung oder Umlagerung eines Nutzers entstehen. Dazu ist das Schwenkelement um mindestens eine Achse gegenüber dem Grundkörper bzw. zumindest einem Teil des Grundkörpers schwenkbar. Bspw. kann das Schwenkelement gegenüber einem Leitelement des Grundkörpers schwenkbar sein. Dazu kann bzw. können der Grundkörper und/oder das Schwenkelement ein Schwenkgelenk aufweisen.

Insbesondere kann das Schwenkelement gegenüber einer Radialachse einer Öffnung des Grundkörpers horizontal und/oder vertikal schwenkbar sein. Dazu kann das Schwenkelement mit dem Grundkörper durch ein Schwenkgelenk, wie bspw. ein Drehgelenk oder ein Kugelgelenk bzw. ein Lager verbunden sein. Das Schwenkgelenk kann eine Öffnung des Grundkörpers mit einer Öffnung des Schwenkelements verbinden, so dass das Schwenkelement um die Öffnung des Grundkörpers herum bzw. an der Öffnung des Grundkörpers entlang gedreht werden kann.

Die Aufnahme ist insbesondere als integraler Bestandteil des Grundkörpers des vorgestellten Konnektors ausgestaltet. Durch die Integration der Aufnahme in den Grundkörper des vorgestellten Konnektors wird ein Totraum eines den Konnektor umfassenden Gesamtsystems minimiert, da durch die räumlich kompakte Zusammenfügung von Aufnahme und Grundkörper ein zu dem Konnektor zusätzliches Volumen an Totraum, wie es bei einer Anordnung eines Sensors mittels eines zusätzlich zu einem Konnektor vorzusehenden Verbindungsmoduls typisch ist, vermieden wird.

Durch die Integration der Aufnahme in den Grundkörper ist der vorgestellte Konnektor besonders kompakt in seinen räumlichen Ausmaßen, so dass eine Hebelwirkung eines Hebels zwischen dem Konnektor und einem Nutzer, die durch eine auf den Konnektor wirkende Zug- und/oder Druckkraft verstärkt wird, minimiert wird.

Die erfindungsgemäß vorgesehene Aufnahme kann bspw. als Messfenster ausgestaltet sein, in das ein Sensor einzubringen bzw. einbringbar ist. Ferner kann die Aufnahme eine Küvette aufweisen, die einen Aufbau umfasst, mit dem ein jeweiliger Sensor verbindbar bzw. zu verbinden ist.

Es ist erfindungsgemäß vorgesehen, dass der Konnektor als Winkelstück ausgestaltet ist und das Schwenkelement abgewinkelt zu dem Grundkörper angeordnet ist. Alternativ kann der Konnektor als lineares Adapterstück ausgestaltet ist und ein durch den Grundkörper und das Schwenkelement strömender Luftstrom lediglich entlang einer horizontalen Achse strömen.

Durch ein abgewinkelt zu dem erfindungsgemäß vorgesehenen Grundkörper angeordnetes Schwenkelement, d.h. einem Schwenkelement, das bspw. ein einem Winkel zwischen 1° und 179°, insbesondere zwischen 1° und 89° zu einer Radialachse einer Öffnung des Grundkörpers angeordnet ist, kann eine besonders ergonomische Ausführung des vorgestellten Konnektors erreicht werden, die Zug- und/oder Druckbelastungen durch einen Nutzer minimiert.

Durch eine lineare Ausgestaltung des vorgestellten Konnektors, bei der ein durch den Grundkörper und das Schwenkelement strömender Luftstrom lediglich entlang einer horizontalen Achse bzw. einer Symmetrieachse strömt, kann ein besonders hoher bzw. starker Volumenstrom bzw. ein geringer Widerstand gegenüber dem durch den Grundkörper und das Schwenkelement strömenden Luftstrom erreicht werden, der sich insbesondere zur Beatmung von Nutzern mit schwacher Atemmuskulatur eignet.

Es kann weiterhin vorgesehen sein, dass der Konnektor mindestens ein Strömungselement aufweist, das dazu konfiguriert ist, einen durch den Konnektor strömenden Luftstrom bis zu einem vorgegebenen Mindestdruck bzw. Mindeststaudruck zu stauen. Dabei kann der Mindestdruck durch eine definierte Kennlinie "Druck über Durchfluss" vorgegeben werden.

Durch ein Strömungselement kann ein durch den Konnektor strömender Luftstrom derart gestaut werden, dass ein Mindestdruck zum Durchführen einer Messung, wie bspw. einer Differenzdruckmessung, insbesondere einer Volumenstrommessung zur Bestimmung eines Atemminutenvolumens oder einer Druckmessung zur Bestimmung eines Atemwegdrucks, erreicht wird. Dazu kann das Strömungselement horizontal oder vertikal in dem Konnektor angeordnet sein und bspw. als Anordnung parallel verlaufender Platten ausgestaltet sein.

Es kann weiterhin vorgesehen sein, dass das Strömungselement zumindest bereichsweise beweglich ist und dazu konfiguriert ist, einen das Strömungselement anströmenden Luftstrom in einen gemäß einer vorgegebenen Verlaufsfunktion strömenden Luftstrom zu überführen.

Durch ein zumindest teilweise bewegliches Strömungselement, wie bspw. eine Strömungsklappe, kann, bspw. unter Verwendung eines Federelements, eine Stärke eines durch das Strömungselement bereitzustellenden Strömungswiderstands vorgegeben bzw. angepasst werden. Dazu kann das Strömungselement selbst aus einem federnden Material, wie bspw. Kunststoff bestehen oder mindestens ein Federelement, wie bspw. eine mechanische Feder umfassen.

Insbesondere kann durch ein bewegliches Strömungselement ein bspw. in Abhängigkeit eines Auslenkungswinkels des Strömungselements veränderlicher Strömungswiderstand bereitgestellt werden, so dass bspw. bei einem kleinen Auslenkungswinkel des Strömungselements ein besonders starker Strömungswiderstand bereitgestellt wird, um einen Mindeststaudruck, der für eine Messung mittels eines Differenzdrucksensors erforderlich ist, bereitzustellen.

Es kann weiterhin vorgesehen sein, dass die mindestens eine Aufnahme mindestens zwei Messfenster bzw. Druckabgriffe, wie bspw. Leitungen, die durch jeweilige Messfenster bzw. Öffnungen strömende Luft zu einem Sensor leiten, umfasst. Zusätzlich können zwei Messfenster zur Kohlenstoffdioxidmessung mittels bspw. Infrarotstrahlung vorgesehen sein.

Mittels einer Aufnahme, in der Druckabgriffe an fest vorgegebenen Positionen vorgesehen sind, kann ein Strömungsfluss zu einem in bzw. an der Aufnahme angeordneten bzw. anzuordnenden Sensor vorgegeben werden, so dass der Sensor entsprechend standardisierte bzw. exakte Messwerte ermittelt.

Der vorgestellte Konnektor bzw. ein an dem Konnektor anzuordnender Sensor dient insbesondere zur Volumenstrommessung zur Bestimmung eines Atemminutenvolumens, zur Druckmessung zur Bestimmung eines Atemwegdrucks oder zur Durchführung einer Messfunktion zur Bestimmung einer Kohlenstoffdioxidkonzentration in einem Atemgas.

Es kann weiterhin vorgesehen sein, dass die mindestens eine Aufnahme starr oder schwenkbar mit dem Schwenkelement des Konnektors verbunden ist.

Durch eine schwenkbare Aufnahme bzw. eine zumindest teilweise schwenkbar ausgestaltete Aufnahme kann ein zu der erfindungsgemäß vorgesehenen schwenkbaren Verbindung zwischen Schwenkelement und Grundkörper zusätzlicher Schwenkbereich bzw. ein zusätzlicher Freiheitsgrad für eine Bewegung des Konnektors relativ zu einem Nutzer vorgesehen werden. Insbesondere kann es vorgesehen sein, dass der Grundkörper um eine erste Achse, wie bspw. eine Symmetrieachse einer Öffnung des Schwenkelements und die Aufnahme bzw. zumindest ein Teil der Aufnahme um eine weitere Achse, wie bspw. eine zu der Symmetrieachse vertikal stehende Achse, schwenkbar ausgestaltet ist.

Erfindungsgemäß ist vorgesehen, dass die mindestens eine Aufnahme in dem mindestens einen Schwenkelement angeordnet ist. Alternativ oder ergänzend kann vorgesehen sein, dass die mindestens eine Aufnahme in dem mindestens einen Grundkörper angeordnet ist.

Es kann weiterhin vorgesehen sein, dass die mindestens eine Aufnahme den mindestens einen Sensor derart an dem Konnektor anordnet, dass dieser zur Durchführung einer Hauptstrommessung geeignet ist.

Mittels der erfindungsgemäß vorgesehenen Aufnahme ist es möglich, eine Position eines in bzw. an der Aufnahme anzuordnenden Sensors in dem Konnektor und, dadurch bedingt, relativ zu weiteren Komponenten eines Gesamtsystems, wie bspw. einem Ventilationsgerät oder einem Tubus, vorzugeben. Insbesondere kann die Aufnahme derart in den Konnektor integriert sein, dass ein in bzw. an der Aufnahme anzuordnender Sensor einen durch den Konnektor strömenden Hauptstrom erfasst und entsprechend analysiert.

Insbesondere kann es vorgesehen sein, dass die mindestens eine Aufnahme an sich an einer vorgegebenen Position in einem Hauptstrom eines Beatmungsgeräts angeordnet ist.

Es kann weiterhin vorgesehen sein, dass das mindestens eine Schwenkelement mit dem mindestens einen Grundkörper derart verbunden ist, dass der Konnektor ein minimales Volumen aufweist.

Der erfindungsgemäße Konnektor eignet sich insbesondere zur räumlichen Integration einer Vielzahl von Elementen eines Beatmungssystems in einem vorgegebenen, vorzugsweise minimalen Raum. Durch die kompakte räumliche Ausgestaltung des erfindungsgemäßen Konnektors ist es möglich, einen Totraum, d.h. den Teil eines Beatmungssystems, der während einer Exspiration und während einer Inspiration mit Atemgas durchströmt wird, zu minimieren. Durch einen minimalen Totraum wird ein Anteil von bereits ausgeatmetem Atemgas in einem Volumen an frischem Atemgas, das einem Nutzer zugeführt wird, minimiert, so dass der Anteil an frischem Atemgas, das dem Nutzer zugeführt wird, maximiert wird.

Es kann weiterhin vorgesehen sein, dass das mindestens eine Schwenkelement mit dem mindestens einen Grundkörper mittels mindestens eines um mindestens eine Achse schwenkbaren Schwenkgelenks direkt verbunden ist.

Ein Drehgelenk, wie bspw. eine Ringkupplung oder ein Kugelgelenk, ermöglicht eine Bewegung des erfindungsgemäß vorgesehenen Grundkörpers relativ zu dem erfindungsgemäß vorgesehenen Schwenkelement, um mindestens eine Achse. Dazu kann das Schwenkgelenk bspw. einen starren und einen beweglichen Teil aufweisen, wobei bspw. der starre Teil mit dem Schwenkelement verbunden sein kann. Insbesondere ist das Schwenkgelenk als integraler Bestandteil des Schwenkelements und/oder des Grundkörpers ausgestaltet.

Es kann weiterhin vorgesehen sein, dass der Grundkörper und/oder das Schwenkelement und/oder die mindestens eine Aufnahme mindestens ein zusätzliches um mindestens eine Achse schwenkbares Schwenkgelenk aufweist bzw. aufweisen.

Durch ein zusätzliches Schwenkgelenk bzw. eine Vielzahl von Schwenkgelenken kann eine Vielzahl von Freiheitsgraden in einer Bewegung des erfindungsgemäßen Konnektors relativ zu einem Nutzer bereitgestellt werden. Insbesondere kann vorgesehen sein, dass jedes Schwenkgelenk einer Vielzahl Schwenkgelenke um eine jeweilige Achse schwenkbar ist, so dass der Konnektor durch die Vielzahl Schwenkgelenke um eine Vielzahl Achsen zu schwenken bzw. schwenkbar ist.

Es kann weiterhin vorgesehen sein, dass in dem Konnektor mindestens ein Strömungselement angeordnet ist.

Es kann weiterhin vorgesehen sein, dass der Konnektor mindestens einen Sensor umfasst und der mindestens eine Sensor zum Ermitteln eines Luftmassenstroms und/oder einer Kohlenstoffdioxidkonzentration eines durch den Konnektor strömenden Luftstroms konfiguriert ist.

Insbesondere kann der mindestens eine Sensor als Differenzdrucksensor ausgestaltet sein. Dazu kann vorgesehen sein, dass die erfindungsgemäß vorgesehene Aufnahme mindestens zwei Messfenster bzw. Druckabgriffe aufweist, durch die jeweils eine Drucksäule eines durch den Konnektor strömenden Luftstroms dem Differenzdrucksensor zuzuführen ist. Anhand der beiden Drucksäulen kann durch den Differenzdrucksensor auf Strömungseigenschaften des durch den Konnektor strömenden Luftstroms geschlossen werden. Die Druckabgriffe können bspw. mit einem Leitungssystem zum Versorgen des Differenzdrucksensors mit Luft verbunden sein, so dass die Druckabgriffe auch zum Anordnen des Differenzdrucksensors an dem Konnektor dienen.

Ferner kann der erfindungsgemäß vorgesehene Sensor dazu konfiguriert sein, einen Luftmassenstrom, d.h. ein Volumen eines durch den Konnektor strömenden Luftstroms pro Zeiteinheit, zu erfassen.

Ferner kann der erfindungsgemäß vorgesehene Sensor dazu konfiguriert sein, eine Kohlenstoffdioxidkonzentration, bspw. in [%] oder in [mg/l] in einem durch den Konnektor strömenden Luftstrom zu erfassen. Dazu kann der Sensor bspw. ein Infrarotelement oder jedes weitere zum Analysieren einer optischen Dichte und/oder eines Gasdrucks geeignete Element, umfassen.

Es kann weiterhin vorgesehen sein, dass der mindestens eine Sensor ein bidirektionaler Sensor ist.

Mittels eines bidirektionalen Sensors kann ein durch den erfindungsgemäßen Konnektor strömendes Gas sowohl beim Strömen in Inspirationsrichtung als auch beim Strömen in Exspirationsrichtung analysiert werden.

Insbesondere dient der erfindungsgemäße Konnektor zur Verwendung bei einer künstlichen Beatmung eines Nutzers, bspw. bei der Behandlung von Atemwegserkrankungen.

Ferner betrifft die vorgestellte Erfindung ein Beatmungssystem zum Beatmen eines Nutzers, wobei das Beatmungssystem eine Ausführungsform des erfindungsgemäßen Konnektors aufweist, und der Konnektor mit einem Ventilationsgerät des Beatmungsgeräts verbunden ist.

Der erfindungsgemäße Konnektor dient insbesondere zum Bereitstellen des erfindungsgemäßen Beatmungssystems.

### BEVORZUGTE AUSFÜHRUNGSBEISPIELE

Weitere, die Erfindung verbessernde Maßnahmen ergeben sich aus der nachfolgenden Beschreibung zu einigen Ausführungsbeispielen der Erfindung, welche in den Figuren dargestellt sind. Sämtliche aus den Ansprüchen, der Beschreibung oder der Zeichnung hervorgehende Merkmale und/oder Vorteile, einschließlich konstruktiver Einzelheiten und räumlicher Anordnungen können sowohl für sich, als auch in den verschiedenen Kombinationen erfindungswesentlich sein. Es zeigen jeweils schematisch:
- Figur 1: eine Seitenansicht einer möglichen Ausgestaltung des Konnektors.
- Figur 2: eine Seitenansicht des Konnektors aus Fig.1 mit einem Strömungselement.
- Figur 3: eine Seitenansicht des Konnektors aus Fig. 1 mit einer Strömungsklappe.
- Figur 4: eine Seitenansicht einer möglichen Ausgestaltung des Konnektors, bei der der Konnektor als lineares Modul ausgestaltet ist.
- Figur 5: eine Seitenansicht einer möglichen Ausgestaltung des erfindungsgemäßen Konnektors mit einer in einem Grundkörper des Konnektors vorgesehenen Aufnahme.
- Figur 6: eine Darstellung einer möglichen Ausgestaltung des erfindungsgemäßen Beatmungssystems.

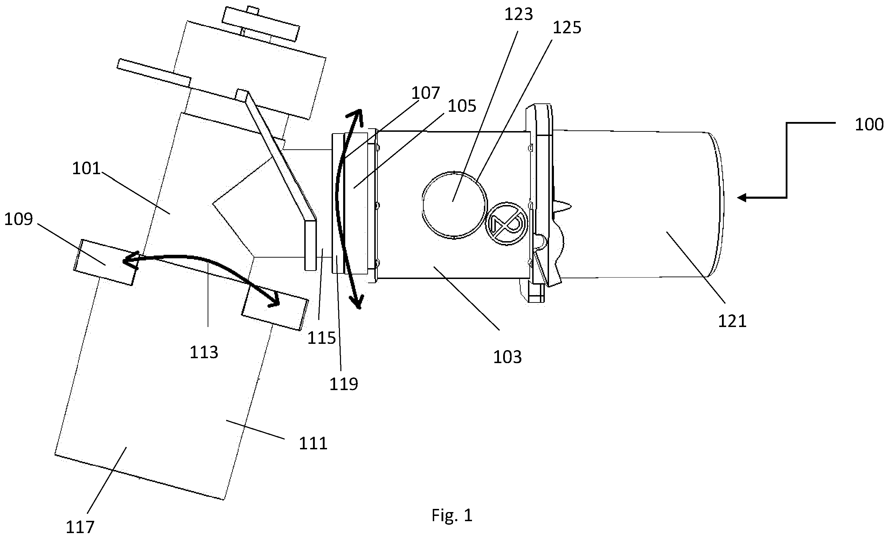

Elemente mit gleicher Funktion und Wirkungsweise sind in den Fig. 1 bis 6 jeweils mit denselben Bezugszeichen versehen.

In Fig. 1 ist ein Konnektor 100 dargestellt. Der Konnektor 100 umfasst ein Schwenkelement 101 und einen Grundkörper 103.

Der Konnektor 100 ist hier als Winkelkonnektor ausgestaltet, so dass ein den Konnektor 100 durchströmender Luftstrom in einem Winkel umgeleitet wird, und nicht entlang einer geraden bzw. horizontalen Ebene durch den Konnektor 100 strömt.

Der Grundkörper 103 ist gegenüber dem Schwenkelement 101 durch ein erstes Schwenkgelenk 105 schwenkbar, d.h. entlang einer horizontalen bzw. im Wesentlichen horizontalen Rotationsachse, wie durch einen ersten Pfeil 107 angedeutet, drehbar angeordnet.

Eine im Wesentlichen horizontale Rotationsachse kann dabei bspw. in einem Bereich zwischen 1° und 25° gegenüber der horizontalen Rotationsachse geneigt sein.

Weiterhin ist das Schwenkelement 101 durch ein zweites Schwenkgelenk 109 gegenüber einem Nutzeranschluss 111 schwenkbar, d.h. entlang einer weiteren Rotationsachse, die bspw. in einem Winkel zu der Horizontalachse liegt, drehbar angeordnet, wie durch einen zweiten Pfeil 113 angedeutet. An dem Nutzeranschluss 111, der als Teil des Schwenkelements 101 ausgestaltet sein kann, kann bspw. ein Tubus, ein Filter oder eine Maske eines Patienten angeschlossen werden.

Durch die beiden Rotationsachsen bzw. die Schwenkgelenke 105 und 109 entlastet der Konnektor 100 ein mit dem Konnektor 100 bspw. über das Anschlussstück 121 verbundenes Ventilationsgerät von einer durch eine Bewegung eines mit dem Schwenkelement 103 über den Nutzeranschluss 111 verbundenen Nutzers vermittelten Zug- bzw. Druckkraft. Mit anderen Worten ermöglicht der Konnektor 100 eine Bewegung des Nutzers entlang der Rotationsachsen gegenüber dem mit dem Konnektor 100 verbundenen Ventilationsgerät, ohne dass dadurch Zug- bzw. Druckkräfte auf das Ventilationsgerät übertragen werden.

Das Schwenkelement 101 ist auf einer ersten Seite 115 direkt mit dem Grundkörper 103 verbunden.

Der Konnektor 100 kann auf einer zweiten Seite 117 über den Nutzeranschluss 111 mit einem Nutzer verbunden werden.

Der Grundkörper 103 wird auf einer dritten Seite 119 direkt mit der ersten Seite 115 des Schwenkelements 101 verbunden.

Zur Verbindung der dritten Seite 119 des Grundkörpers 103 mit der ersten Seite 115 des Schwenkelements 101 ist hier das Schwenkgelenk 105 vorgesehen. Das Schwenkgelenk 105 kann komplett als Teil des Schwenkelements 101 ausgestaltet sein oder mit einem ersten Teil als Teil des Grundkörpers 103 und mit einem zweiten Teil als Teil des Schwenkelements 101 ausgestaltet sein. Alternativ kann das erste Schwenkgelenk 105 als Teil des Grundkörpers 103 ausgestaltet sein.

Der Grundkörper 103 wird auf einer vierten Seite 121 mit einem Ventilationsgerät verbunden.

Weiterhin umfasst der Grundkörper 103 eine Aufnahme 125, mittels derer ein Sensor an dem Konnektor 100 anzuordnen ist, um einen durch den Konnektor 100 strömenden Luftstrom zu analysieren. Die Aufnahme 125 dient hier zum Anordnen eines optischen Sensors, wie bspw. einem Infrarotsensor zum Erfassen von bspw. einer Kohlenstoffmonoxid- und/oder einer Kohlenstoffdioxidkonzentration. Dazu umfasst die Aufnahme 125 ein transparentes Element 123, das für Strahlen des optischen Sensors durchlässig ist.

Durch die fest in dem Grundkörper 103 vorgegebene Position der Aufnahme 125 und die fest vorgegebene Position des Konnektors 100 in einem jeweiligen Beatmungssystem kann der Sensor lediglich an einer fest vorgegebenen Position in bzw. relativ zu einem jeweiligen Ventilationsgerät bzw. Nutzer in dem Beatmungssystem angeordnet werden. Entsprechend werden positionsbedingte Messfehler des Sensors, die bspw. durch eine bzgl. einer Kalibrierungslänge bzw. einer ursprünglichen Länge längere oder kürzere Messstrecke eines zu messenden Atemgases bedingt sind, minimiert bzw. vermieden.

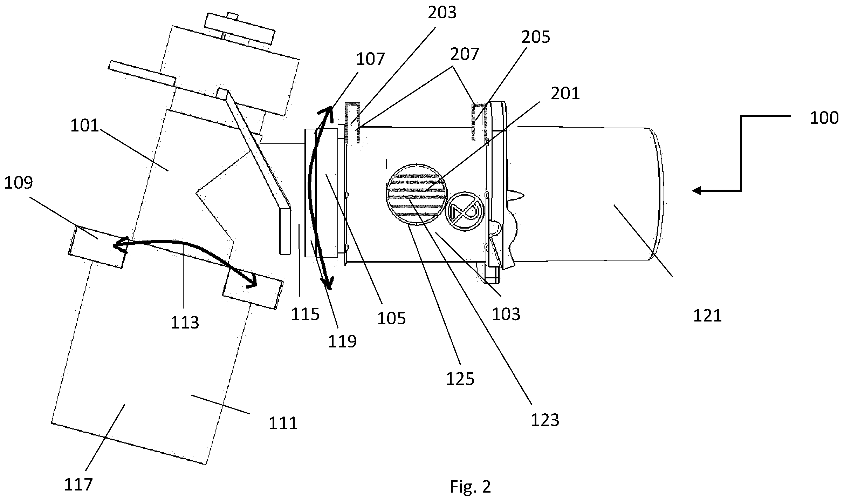

In Fig. 2 ist der Konnektor 100 mit einem Strömungselement 201 dargestellt, durch das durch den Konnektor 100 strömende Luft in dem Konnektor 100 gestaut wird, so dass sich beim Betrieb des Konnektors 100 ein vorgegebener Mindeststaudruck einstellt und ein an dem Konnektor 100 angeordneter Sensor, wie bspw. ein Differenzdrucksensor, zum Erfassen von Strömungseigenschaften des durch den Konnektor 100 strömenden Luftstroms verwendet werden kann.

Das in Fig. 2 dargestellte Strömungselement 201 verläuft durch den Grundkörper 103, insbesondere entlang einer gesamten Länge des Grundkörpers 103 und ist in Fig. 2 nur in einem durch das transparente Element 123 hindurch sichtbaren Bereich dargestellt.

Das Strömungselement 201 kann zum Aufbauen von Staudruck für einen Luftstrom konfiguriert sein, der von einer Nutzerseite bzw. der ersten Seite 115 aus durch den Konnektor 100 strömt. Alternativ oder zusätzlich kann das Strömungselement 201 zum Aufbauen von Staudruck für einen Luftstrom konfiguriert sein, der von einer Geräteseite bzw. der vierten Seite 121 aus durch den Konnektor 100 strömt, so dass Strömungseigenschaften eines Nutzerluftstroms und/oder eines Geräteluftstroms ermittelt werden können.

Ferner umfasst der in Fig. 2 dargestellte Grundkörper 103 eine zweite Aufnahme 207 zum Anordnen eines Sensors. Die zweite Aufnahme 207 umfasst einen ersten Druckabgriff 203 an einem ersten Messfenster und ein benachbart zu dem ersten Druckabgriff 203 angeordneten zweiten Druckabgriff 205 an einem zweiten Messfenster 205. Der erste Druckabgriff 203 und der zweite Druckabgriff 205 sind über ein nicht dargestelltes Verbindungssystem mit dem Sensor verbunden, wodurch der Sensor mit dem Konnektor 100 verbunden bzw. an diesem derart angeordnet wird, dass durch den ersten Druckabgriff 203 und den zweiten Druckabgriff 205 strömende Luft zu dem Sensor geleitet wird, und der Sensor, der bspw. ein Differenzdrucksensor sein kann, anhand der entsprechenden Luftdrücke bzw. Differenzdrücke zwischen jeweiligen Drucksäulen, Strömungseigenschaften eines durch den Konnektor 100 strömenden Luftstroms bestimmen kann.

Durch das Strömungselement 201 wird im Bereich des ersten Druckabgriffs 203 und des zweiten Druckabgriffs 205 ein Staudruck erzeugt, der bewirkt, dass durch den Konnektor 100 strömende Luft zumindest teilweise zu dem Sensor strömt und der Sensor Eigenschaften des durch den Konnektor 100 strömenden Luftstroms erfassen kann. Dabei ist das Strömungselement 201 derart gestaltet, dass der Sensor zumindest mit einem für eine Messung erforderlichen Minimaldruck versorgt wird und ein Widerstand für den durch den Konnektor 100 strömenden Luftstrom minimal bleibt. Dazu kann das Strömungselement 201 bspw. als Anordnung von insbesondere parallel angeordneten Platten ausgestaltet sein, wie es in Fig. 2 dargestellt ist.

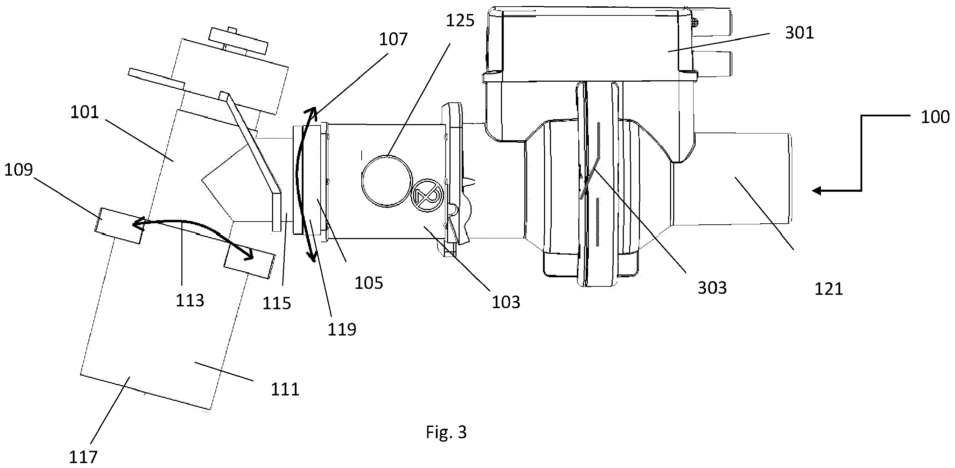

In Fig. 3 umfasst der Konnektor 100 ein Strömungselement 303 in Form einer Strömungsklappe und eine Aufnahme 301 zum Anordnen eines Differenzdrucksensors an dem Konnektor 100 mittels eines Verbindungssystems, wie bspw. einer Anzahl Messleitungen.

Hier kann die Aufnahme 301 bspw. einen ersten Druckabgriff 203 und einen zweiten Druckabgriff 205, wie in Fig. 2 dargestellt, umfassen. Selbstverständlich kann die Aufnahme 301 auch jeden weiteren technisch geeigneten Druckabgriff zum Bereitstellen von Luftsäulen für eine Differenzdruckmessung umfassen.

Das Strömungselement 303 ist hier als teilweise beweglich in dem Konnektor 100 angeordnete Strömungsklappe ausgestaltet, so dass das Strömungselement 303 sich in Abhängigkeit eines durch den Konnektor 100 strömenden Luftstroms bewegt. Dies bedeutet, dass das Strömungselement 303 sich bei einem von der ersten Seite 115 aus durch den Konnektor 100 strömenden Luftstrom von dem Schwenkelement 101 weg und sich bei einem von der vierten Seite 121 durch den Konnektor 100 strömenden Luftstrom zu dem Schwenkelement 101 hin bewegt. Dabei bewegt sich das Strömungselement 303 in Abhängigkeit eines Drucks eines jeweilig anströmenden Luftstroms, so dass ein hinter dem Strömungselement 303 strömender Luftstrom mit einer gemäß einer vorgegebenen Kennlinie strömenden Strömungscharakteristik strömt. Entsprechend ermöglicht das Strömungselement einen Abgriff sowohl eines inspiratorischen Drucks als auch eines exspiratorischen Drucks.

Insbesondere kann das Strömungselement 303 derart ausgestaltet sein, dass dieses einen mit einem geringen Druck durch den Konnektor 100 strömenden Luftstrom stärker staut als einen mit einem hohen Druck strömenden Luftstrom, so dass der mit geringem Druck strömende Luftstrom zu einem Mindestdruck aufgestaut wird und, dadurch bedingt, eine Weiterführung des Drucks zu einem Drucksensor ermöglicht.

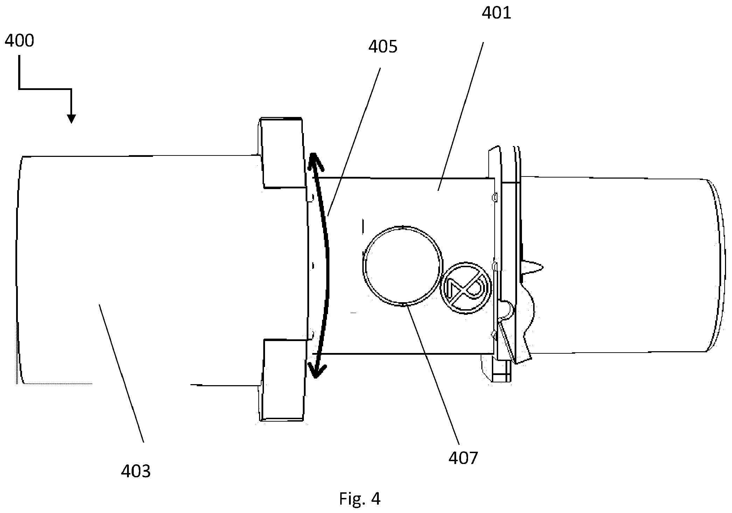

In Fig. 4 ist ein Konnektor 400 dargestellt. Der Konnektor 400 ist hier als lineares Modul bzw. Linearkonnektor ausgestaltet, so dass ein den Konnektor 400 durchströmender Luftstrom nicht in einem Winkel umgeleitet wird, und entsprechend entlang einer geraden bzw. horizontalen Ebene durch den Konnektor 400 strömt. Dabei ist ein Grundkörper 401 des Konnektors 400 gegenüber einem Schwenkelement 403 analog zu den in Figuren 1 bis 3 beschriebenen Mechanismen drehbar angeordnet, wie es durch Pfeil 405 angedeutet ist. Dazu kann ein Schwenkgelenk an dem Grundkörper 401 oder dem Schwenkelement 403 angeordnet sein.

Hier kann bspw. an einer Aufnahme 407 ein Sensor angeordnet werden, wie es bereits mit Bezug auf Figuren 1 bis 3 beschrieben ist.

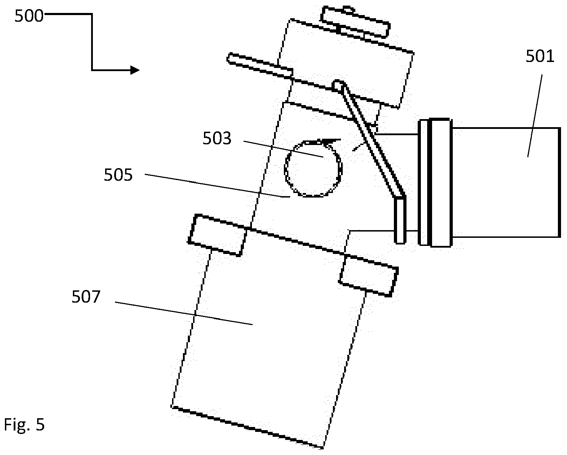

In Fig. 5 ist ein erfindungsgemäßer Konnektor 500 dargestellt, dessen Schwenkelement 505 eine Aufnahme 503 zum Anordnen eines Sensors an dem Konnektor 500 aufweist. Durch die in das Schwenkelement 505 integrierte Aufnahme 503 kann ein in der Aufnahme 503 angeordneter Sensor in besonders kurzer Distanz zu einem Nutzer angeordnet werden kann, wodurch ein Totraum eines entsprechenden Beatmungssystems minimiert wird. Das Schwenkelement 505 ist hier drehbar bzw. schwenkbar gegenüber einem Grundkörper 501 angeordnet, wie es analog bereits mit Bezug auf Figuren 1 bis 3 beschrieben ist. Dazu ist das Schwenkelement 505 über ein Schwenkgelenk des Grundkörpers 501 oder ein Schwenkgelenk des Schwenkelements 505 direkt und schwenkbar, d.h. entlang einer Rotationsachse drehbar, an dem Grundkörper 501 angeordnet.

Weiterhin ist das Schwenkelement 505 durch ein zweites Schwenkgelenk gegenüber einem Nutzeranschluss 507 schwenkbar, d.h. entlang einer weiteren Rotationsachse drehbar, angeordnet. An dem Nutzeranschluss 507 kann bspw. ein Tubus, ein Filter oder eine Maske eines Patienten angeschlossen werden.

Durch die beiden Rotationsachsen bzw. die Schwenkgelenke entlastet der Konnektor 500 einen an der Aufnahme 503 angeordneten Sensor von einer durch eine Bewegung eines mit dem Schwenkelement 505 über den Nutzeranschluss 507 verbundenen Nutzers vermittelten Zug- bzw. Druckkraft, so dass auf den Sensor wirkende Zug- bzw. Druckkräfte minimiert werden.

Hier kann bspw. an der Aufnahme 503 ein Sensor angeordnet werden, wie es bereits mit Bezug auf Aufnahme 125 gemäß Fig. 1 beschrieben ist.

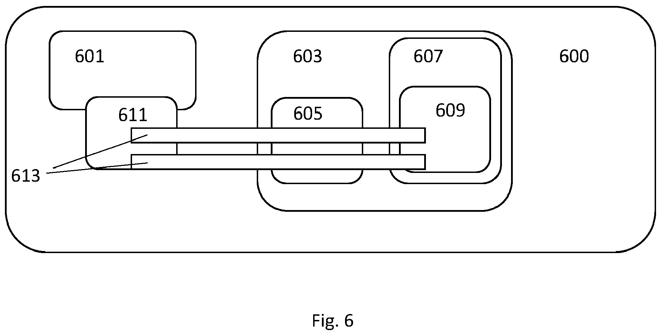

Fig. 6 zeigt ein Beatmungssystem 600. Das Beatmungssystem 600 umfasst ein Ventilationsgerät 601, einen Konnektor 603 mit einem Grundkörper 605, einem Schwenkelement 607 und einer in dem Schwenkelement 607 vorgesehenen Aufnahme 609. An der Aufnahme 609 ist ein Sensor 611 zum Analysieren von durch das Beatmungssystem strömendem Atemgas über ein Verbindungssystem 613 des Sensors 611 angeordnet. Entsprechend verbindet das Verbindungssystem 613 eine Messeinheit des Sensors 611 mit der Aufnahme 609. Dabei umfasst das Verbindungssystem 613 zwei Leitungen, durch die jeweils eine Drucksäule aus dem Konnektor 603 zu der Messeinheit des Sensors 611 geleitet wird, so dass die Messeinheit Eigenschaften eines durch den Konnektor 603 strömenden Luftstroms ermitteln kann.

Das Schwenkelement 607 ist mit einem Nutzer zu verbinden bzw. verbindbar. Die Position der Druckabgriffe des Sensors 611 ist in dem Beatmungssystem 600 durch die Position der Aufnahme 609 fest vorgegeben, so dass bspw. bei einem Wechsel des Sensors 611 oder einem Wechsel des Ventilationsgeräts 601 ein Strömungsweg eines durch das Beatmungssystem 600 strömenden Luftstroms von dem Ventilationsgerät 601 zu dem Sensor 611 bzw. von einem Nutzer zu dem Sensor 611 konstant bleibt. Entsprechend werden Messfehler bzw. Messwertverschiebungen von durch den Sensor 611 ermittelten Messwerten durch positionsbedingte Strömungsunterschiede unter Verwendung des Konnektors 603 vermieden.

### BEZUGSZEICHENLISTE

- 100: Konnektor
- 101: Schwenkelement
- 103: Grundkörper
- 105: erstes Schwenkgelenk
- 107: erster Pfeil
- 109: zweites Schwenkgelenk
- 111: Nutzeranschluss
- 113: zweiter Pfeil
- 115: erste Seite
- 117: zweite Seite
- 119: dritte Seite
- 121: vierte Seite
- 123: transparentes Element
- 125: Aufnahme
- 201: lineares Strömungselement
- 203: erster Druckabgriff
- 205: zweiter Druckabgriff
- 207: zweite Aufnahme
- 301: Aufnahme
- 303: Strömungselement
- 400: Konnektor
- 401: Grundkörper
- 403: Schwenkelement
- 405: Pfeil
- 407: Aufnahme
- 500: Konnektor
- 501: Grundkörper
- 503: Aufnahme
- 505: Schwenkelement
- 507: Nutzeranschluss
- 600: Beatmungssystem
- 601: Ventilationsgerät
- 603: Konnektor
- 605: Grundkörper
- 607: Schwenkelement
- 609: Aufnahme
- 611: Sensor
- 613: Verbindungssystem

## Patentansprüche

1. Konnektor (100, 400, 500, 603) zum Anordnen mindestens eines Sensors (611) an einem Beatmungssystem (600),
wobei der Konnektor (100, 400, 500, 603) aufweist:
- mindestens einen Grundkörper (103, 401, 501, 605) zur Verbindung mit mindestens einem Ventilationsgerät (601),
- mindestens ein Schwenkelement (101, 403, 505, 607) zur Verbindung mit einem Nutzer, und
- mindestens eine Aufnahme (125, 207, 301, 407, 503, 609) für mindestens einen Sensor (611),
wobei das mindestens eine Schwenkelement (101, 403, 505, 607) auf einer ersten Seite (119) direkt und nicht lösbar mit dem Grundkörper (103, 401, 501, 605) verbunden ist, und
wobei das mindestens eine Schwenkelement (101, 403, 505, 607) um mindestens eine Achse gegenüber zumindest einem Teil des Grundkörpers (103, 401, 501, 605) schwenkbar ist, um den Grundkörper (103, 501, 605) gegenüber durch den Nutzer vermittelten Zug- bzw. Druckkräften zu schützen
**dadurch gekennzeichnet,**
**dass** der Konnektor (100, 400, 500, 603) als Winkelstück ausgestaltet ist und der Grundkörper (103, 501, 605) abgewinkelt zu dem Schwenkelement (101, 403, 505, 607) angeordnet ist, und
**dass** die mindestens eine Aufnahme (125, 207, 301, 407, 503, 609) an dem mindestens einen Schwenkelement (103, 401, 501, 605) angeordnet ist.

2. Konnektor (100, 400, 500, 603) nach Anspruch 1,
**dadurch gekennzeichnet, dass**
der Konnektor (100, 400, 500, 603) mindestens ein Strömungselement (201, 303) aufweist, das dazu konfiguriert ist, einen durch den Konnektor (100, 400, 500, 603) strömenden Luftstrom bis zu einem vorgegebenen Mindestdruck zu stauen.

3. Konnektor (100, 400, 500, 603) nach Anspruch 2,
**dadurch gekennzeichnet, dass**
das Strömungselement (201, 303) zumindest bereichsweise beweglich ist und dazu konfiguriert ist, einen das Strömungselement (201, 303) anströmenden Luftstrom in einen gemäß einer vorgegebenen Verlaufsfunktion strömenden Luftstrom zu überführen.

4. Konnektor (100, 400, 500, 603) nach einem der vorangehenden Ansprüche, **dadurch gekennzeichnet, dass**
das Strömungselement (201, 303) horizontal oder vertikal verlaufend in dem Konnektor (100, 400, 500, 603) angeordnet ist.

5. Konnektor (100, 400, 500, 603) nach einem der voranstehenden Ansprüche, **dadurch gekennzeichnet, dass**
die mindestens eine Aufnahme (125, 207, 301, 407, 503, 609) mindestens zwei Druckabgriffe (203, 205) zum Ermitteln eines Differenzdrucks in einem durch den Konnektor (100, 400, 500, 603) strömenden Luftstrom umfasst.

6. Konnektor (100, 400, 500, 603) nach einem der voranstehenden Ansprüche, **dadurch gekennzeichnet, dass**
die mindestens eine Aufnahme (125, 207, 301, 407, 503, 609) starr oder schwenkbar mit dem mindestens einen Grundkörper (103, 401, 501, 605) des Konnektors (100, 400, 500, 603) verbunden ist.

7. Konnektor (100, 400, 500, 603) nach einem der vorangehenden Ansprüche, **dadurch gekennzeichnet, dass**
die mindestens eine Aufnahme (125, 207, 301, 407, 503, 609) dazu konfiguriert ist, den mindestens einen Sensor (611) derart an dem Konnektor (100, 400, 500, 603) anzuordnen, dass dieser zur Durchführung einer Hauptstrommessung geeignet ist.

8. Konnektor (100, 400, 500, 603) nach einem der vorangehenden Ansprüche, **dadurch gekennzeichnet, dass**
das mindestens eine Schwenkelement (101, 403, 505, 607) mit dem mindestens einen Grundkörper (103, 401, 501, 605) derart verbunden ist, dass der Konnektor (100, 400, 500, 603) ein minimales Durchströmungsvolumen aufweist.

9. Konnektor (100, 400, 500, 603) nach einem der vorangehenden Ansprüche, **dadurch gekennzeichnet, dass**
das mindestens eine Schwenkelement (101, 403, 505, 607) mit dem mindestens einen Grundkörper (103, 401, 501, 605) mittels mindestens eines um mindestens eine Achse schwenkbaren Schwenkgelenks (105, 109) direkt verbunden ist.

10. Konnektor (100, 400, 500, 603) nach einem der voranstehenden Ansprüche, **dadurch gekennzeichnet, dass**
das mindestens eine Schwenkelement (101, 403, 505, 607) und/oder der mindestens eine Grundkörper (103, 401, 501, 605) und/oder die mindestens eine Aufnahme (125, 207, 301, 407, 503, 609) mindestens ein zusätzliches um mindestens eine Achse schwenkbares Schwenkgelenk aufweist bzw. aufweisen.

11. Konnektor (100, 400, 500, 603) nach einem der voranstehenden Ansprüche, **dadurch gekennzeichnet, dass**
der Konnektor (100, 400, 500, 603) mindestens einen Sensor (611) aufweist, und der mindestens eine Sensor (611) zum Ermitteln eines Luftmassenstroms und/oder einer Kohlenstoffdioxidkonzentration eines durch den Konnektor (100, 400, 500, 603) strömenden Luftstroms konfiguriert ist.

12. Konnektor (100, 400, 500, 603) nach Anspruch 11,
**dadurch gekennzeichnet, dass**
der mindestens eine Sensor (611) ein bidirektionaler Sensor und/oder ein optischer Sensor ist.

13. Beatmungssystem (600) zum Beatmen eines Nutzers,
wobei das Beatmungssystem (600) einen Konnektor (100, 400, 500, 603) nach einem der Ansprüche 1 bis 12 aufweist, und
wobei der Konnektor (100, 400, 500, 603) mit einem Ventilationsgerät (601) des Beatmungsgeräts (600) verbunden ist.
